# EUROPÄISCHE PATENTSCHRIFT

(11) **EP 0 753 058 B1**
(45) Veröffentlichungstag und Bekanntmachung des Hinweises auf die Patenterteilung: **16.06.2004**
(21) Anmeldenummer: 95913164.0
(22) Anmeldetag: 27.03.1995
(51) Int. Cl.: C12N 9/54, C11D 3/386, C14C 1/00

(54) **HOCHALKALISCHE PROTEASE UND DEREN VERWENDUNG**
HIGH-ALKALINE PROTEASE AND ITS USE
PROTEASE HAUTEMENT ALCALINE ET SON UTILISATION

(30) Priorität: 31.03.1994 DE 4411223
(43) Veröffentlichungstag der Anmeldung: 15.01.1997
(73) Patentinhaber: GENENCOR INTERNATIONAL, INC., Palo Alto, California 94304 (US)
(72) Erfinder: AMORY, Antoine, 1330 Rixensart (BE); CLIPPE, André, 1120 Brüssel (BE); KONIECZNY-JANDA, Gerhard, 30982 Pattensen (DE); HERRMANN, Hubert, 31582 Nienburg (Weser) (DE); BÖRNER, Karsten, 31319 Sehnde (DE)
(74) Vertreter: Armitage, Ian Michael
(86) Internationale Anmeldenummer: PCT/EP1995/001141
(87) Internationale Veröffentlichungsnummer: WO 1995/027049

(56) Entgegenhaltungen:
- EP-A- 0 328 229
- EP-A- 0 415 296
- EP-A- 0 503 346
- WO-A-91/02792
- DE-A- 1 800 508
- DE-A- 4 411 223
- PROTEIN ENGINEERING, Bd. 4, Nr. 7, 1991 OXFORD, Seiten 719-737, SIEZEN, R.J. ET AL 'Homology modeling and protein engineering strategy of subtilases, the family of subtilisin-like serine proteinases'

## Beschreibung

Die Erfindung betrifft eine neue hochalkalische Protease, deren Verwendung im gewerblichen bzw. industriellen Bereich und im Haushaltsbereich sowie auch Zusammensetzungen für die genannten Anwendungen, die diese Protease enthalten.

Der Einsatz proteasehaltiger Zusammensetzungen in gewerblichen oder industriellen Anwendungen bzw. Verfahren, ist bekannt. Beispielsweise werden in gewerblichen Wäschereien, z. B. zur Reinigung von mit Blut verunreinigter Krankenhauswäsche oder von Schützbekleidung aus fleischverarbeitenden Betrieben, bereits seit längerem Proteasen eingesetzt. Im Bereich der Lederbehandlung wird z. B. bei der Lederherstellung in der gegenwärtigen Praxis die Enthaarung der Felle und Häute in einer alkalischen Verfahrensstufe, dem sogenannten Äscher, der die Voraussetzungen für die Enthaarung schafft und den notwendigen Hautaufschluß bewirkt, immer noch unter Verwendung von zum Teil bedenklichen Chemikalien (z. B. anorganische Sulfide) vorgenommen. Zwar wurden in jüngerer Zeit auch bereits enzymatisch gestützte Äscherverfahren (Enthaarungsverfahren) vorgeschlagen - insbesondere unter Verwendung tryptischer Enzyme oder Pilz- oder Bakterienproteasen sowie zum Teil auch Carbohydrolasen, jedoch haben diese enzymatischen Enthaarungsverfahren allerdings fast nur bei Kleintierfellen und auch da nur in beschränktem Maßstab praktische Anwendung erfahren. Bei Großviehhäuten hat sich dagegen die enzymatische Enthaarung bisher nicht durchsetzen können, in erster Linie wegen der zum Teil unvollkommenen Enthaarungswirkung und wegen der Schädigung der Kollagen-Narbenmembran bzw. wegen zu starkem Hautsubstanzabbau. Auch die anteilige Verwendung alkalischer Proteasen im Äscher zusammen mit verminderten Mengen an Chemikalien (z. B. Sulfiden) wurde zur Verminderung der Umweltbelastung durch Chemikalien versucht. Zwar kann der Chemikalienanteil (z. B. Sulfid) durch die Enzymverwendung deutlich gesenkt werden und es werden sehr gute Flächenausbeuten mit wenig Narbenzug erzielt, jedoch tendieren die derart hergestellten Leder zur Losnarbigkeit, zu loser Flämmenstruktur und zu groben, zum Teil nubukierten Narbenbildern. Soweit heute bereits Proteasen im Bereich der Lederherstellung (Hauptweiche und Äscher) eingesetzt werden, besitzen diese Proteasen des Standes der Technik immer noch eine nur geringe Wirksamkeit bei hohen pH-Werten (pH = 11 bis 13) bzw. nur eine relataiv niedige Aktivität bei den in der Wasserwerkstatt üblichen Behandlungstemperaturen (28 bis 30°C).

Aufgrund der in gewerblichen bzw. industriellen Verfahren im Vergleich zu Anwendungen im Haushalt (z. B. als Haushaltswaschmittel) z. T. drastischeren Bedingungen werden an die hierbei verwendeten Proteasen besonders hohe Anforderungen in bezug auf Stabilität, Millieuakzeptanz und Leistung gestellt. Neben einer guten Beständigkeit und Aktivität bei hochalkalischen pH-Werten sollten die Proteasen einerseits auch über eine gute Temperaturstabilität verfügen, um bei niedriger Konzentration für eine möglichst lange Zeitdauer bei den in gewerblichen/industriellen Verfahren zum Teil hohen Temperaturen gute Ergebnisse für den jeweiligen Anwendungszweck zu erbringen, andererseits aber für bestimmte Anwendungen (z.B. Lederherstellung) auch bei relativ niedrigen Temperaturen (etwa 30°C) noch gut Aktivität zeigen. Außerdem sollten die eingesetzten alkalischen Proteasen möglichst unempfindlich gegen die in gewerblichen bzw. industriellen Verfahren üblichen Chemikalien und Inhaltsstoffe (z. B. Tenside, Bleichmittel oder desinfizierend wirkende Substanzen, Chemikalien u. a. Bestandteile) sein. Es besteht daher immer noch ein Bedarf nach weiteren für gewerbliche bzw. industrielle Anwendungen geeigneten alkalischen Proteasen, z. B. für gewerbliche Textilwaschverfahren, gewerbliche Oberflächenreinigung oder die Lederbehandlung bzw. Lederherstellung.

Es bestand daher die Aufgabe, eine neue, insbesondere für die Verwendung in gewerblichen bzw. industriellen Verfahren geeignete, alkalische Protease aufzufinden, die darüber hinaus möglichst aber auch Vorteile beim Einsatz im Haushaltsbereich aufweisen sollte.

Es wurde nun gefunden, daß sich die nachstehend angegebene alkalische Bacillus-Protease mit hoher Wirksamkeit in verschiedenen gewerblichen bzw. industriellen Verfahren einsetzen läßt. Ein Gegenstand der Erfindung ist daher eine alkalische Protease bzw. deren Verwendung, insbesondere in Zusammensetzungen für gewerbliche und industrielle Verfahren bzw. auch im Haushaltsbereich, wie dieses in den Ansprüchen angegeben und nachfolgend näher erläutert ist.

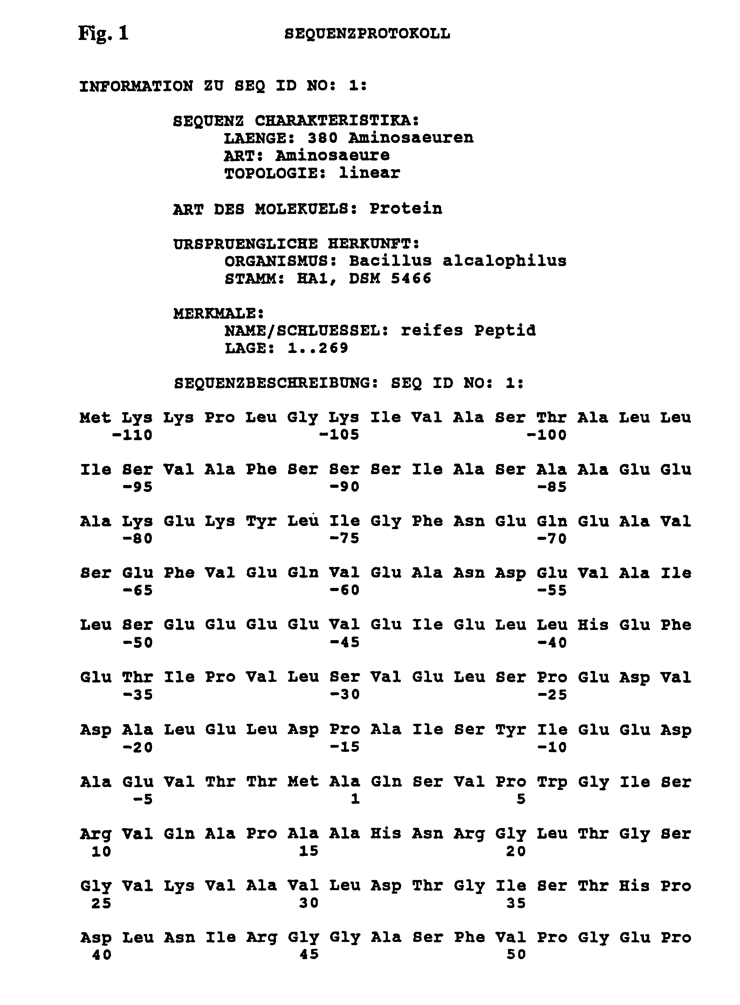

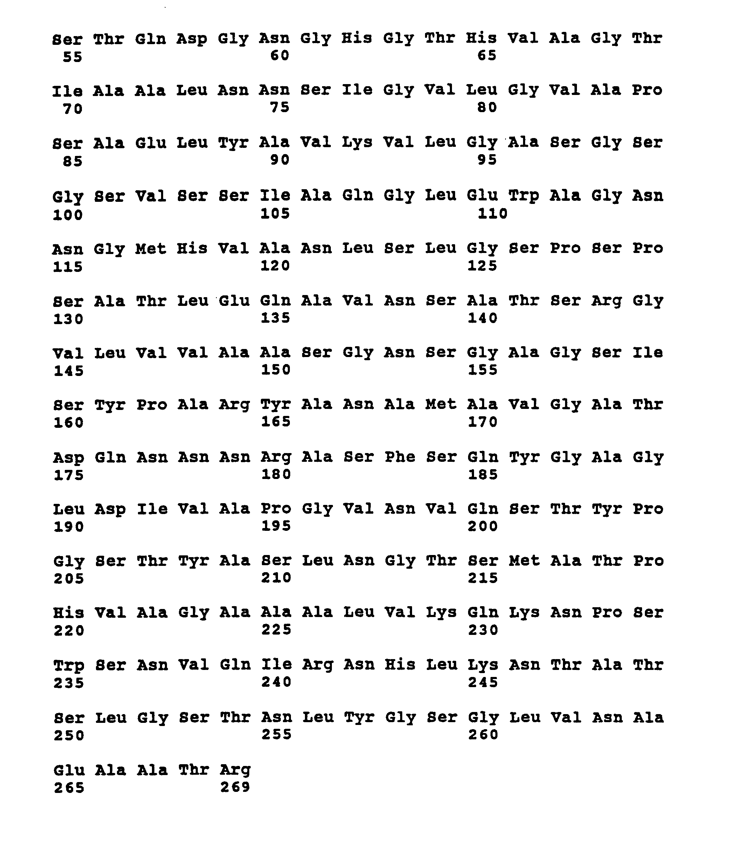

Die Erfindung betrifft demnach eine hochalkalische Protease, die sich dadurch auszeichnet, daß sie eine zugrundeliegende Aminosäurensequenz mit mindestens 95 %, vorzugsweise mit mindestens 98 %, Homologie zu der in Fig. 1 angegebenen Aminosäurensequenz aufweist und sich von dieser durch Dreifachmutation in den Positionen 42/114/115 der Fig. 1 oder in den drei jeweils dazu homologen Positionen dadurch unterscheidet, daß die in den betreffenden Positionen befindlichen Aminosäuren gegen Arginin ausgetauscht sind.

Die genannte alkalische Bacillus-Protease besitzt ein Molekulargewicht von etwa 26.000 bis 28.000 g/mol, gemessen durch SDS-Polyacrylamid-Gelelektrophorese gegenüber Referenzproteinen mit bekanntem Molekulargewicht. Das im analytischen Test mit löslichen Modelsubstraten ermittelte pH-Optimum liegt bei etwa pH 10,5, wobei unter pH-Optimum derjenige pH-Bereich verstanden wird, in dem die Protease maximale proteolytische Aktivität aufweist. Die pH-Aktivität ist hierbei höher als bei der Ausgangsprotease (gemäß Fig. 1); das Wirkoptimum erstreckt sich weiter in den alkalischeren Bereich und liegt bei pH 10,5 bis 11,5. Auch besitzt die genannte erfindungsgemäße alkalische Bacillus-Protease eine gute pH- und Temperatur-Stabilität. Es handelt sich also insbesondere um eine sehr hochalkalische Protease, die in einem pH-Bereich wirksam ist, der durch die bisherigen Proteasen des Standes der Technik nicht erreicht wird.

Unter Homologie zu der in Fig. 1 angegebenen Aminosäurensequenz wird hier die strukturelle Verwandtschaft der betreffenden Aminosäurensequenzen zu der in Fig. 1 angegebenen Aminosäurensequenz verstanden. Zur Bestimmung der Homologie werden jeweils die einander strukturell entsprechenden Abschnitte der Aminosäurensequenz der Fig. 1 und der damit zu vergleichenden Aminosäurensequenz so zur Deckung miteinander gebracht, daß maximale Strukturübereinstimmung zwischen den Aminosäurensequenzen besteht, wobei durch Deletion oder Insertion einzelner Aminosäuren verursachte Unterschiede berücksichtigt und durch entsprechende Verschiebungen von Sequenzabschnitten ausgeglichen werden. Die Zahl der nunmehr in den Sequenzen miteinander übereinstimmenden Aminosäuren ("homologe Positionen") bezogen auf die Gesamtzahl der in der Sequenz der Fig. 1 enthaltenen Aminosäuren gibt dabei die Homologie in % an. Abweichungen in den Sequenzen können sowohl durch Variation, Insertion als auch Deletion von Aminosäuren bedingt sein. Es versteht sich dementsprechend von selbst, daß bei Einsatz von zur Fig. 1 wenigstens zu 95 % homologen alkalischen Proteasen, die mit bezug auf die Fig. 1 bezeichneten Aminosäurenpositionen sich auf die dazu homologen Positionen der jeweils eingesetzten Protease beziehen. Deletionen oder Insertionen in den Aminosäurensequenzen der zu Fig. 1 homologen Proteasen können zu einer relativen Verschiebung der Aminosäurenpositionen führen, so daß in homologen Teilstücken von zueinander homologen Aminosäurensequenzen die numerischen Bezeichnungen der einander entsprechenden Aminosäurenpositionen nicht identisch zu sein brauchen, d. h. es können geringfügige Zahlenverschiebungen bezogen auf die jeweils individuelle Numerierung der Aminosäurenpositionen entstehen.

In einer bevorzugten Variante der Erfindung liegt eine hochalkalische Protease vor, die sich dadurch auszeichnet, daß sie eine zugrundeliegende, im wesentlichen identische Aminosäurensequenz zu der in Fig. 1 angegebenen Aminosäurensequenz aufweist und sich von dieser durch Dreifachmutation in den Positionen 42/114/115 der Fig. 1 dadurch unterscheidet, daß die in den betreffenden Positionen befindlichen Aminosäuren gegen Arginin ausgetauscht sind. Der Begriff "im wesentlichen identische" Aminosäurensequenz bedeutet hierbei, daß mit Ausnahme der genannten Dreifachmutation N42R/N114R/N115R nur einige wenige weitere, d. h. insbesondere nur bis 6 (entspricht in etwa 98 % Homologie oder höher), Aminosäuren von den in der Sequenz der Fig. 1 angegebenen Aminosäuren abweichen können. Die der erfindungsgemäßen hochalkalischen Protease zugrundeliegende Aminosäurensequenz entspricht daher im wesentlichen solchen Proteasen, die als "Subtilisin 309"-Typ bezeichnet werden können, da das im Stand der Technik bekannte "Subtilisin 309" in seiner Aminosäurensequenz identisch mit der in Fig. 1 angegebenen Aminosäurensequenz ist. Eine hierzu fast identische Protease, die mit Ausnahme der Position 85 mit der Aminosäurensequenz in Fig. 1 übereinstimmt, wird im Stand der Technik als Protease aus Bacillus nov. species PB92 (siehe europäische Patentanmeldung EP 283 075) beschrieben. Die Protease aus Bacillus PB92 unterscheidet sich von der in Fig. 1 angegebenen Aminosäurensequenz nur unwesentlich dadurch, daß in Position 85 die natürliche Abweichung Asparagin statt Serin vorhanden ist, und sie wird hier demgemäß als "Subtilisin 309"-Typ-Protease betrachtet. Eine weitere zum "Subtilisin 309" im wesentlichen identische Protease, die mit Ausnahme der fünf Positionen 97, 99, 101, 102 und 157 mit der Aminosäurensequenz in Fig. 1 übereinstimmt, wird im Stand der Technik als Protease aus Bacillus lentus (siehe WO 91/02792 und US 5,352,604) beschrieben ("BLAP"). Die Protease "BLAP" aus Bacillus lentus unterscheidet sich von der in Fig. 1 angegebenen Aminosäurensequenz nur unwesentlich dadurch, daß in fünf Positionen natürliche Abweichungen vorhanden sind: 97D, 99R, 101A, 102I und 157S. Eine Variante von "BLAP" unterscheidet sich zusätzlich durch eine sechste natürliche Abweichung Threonin statt Serin in Position 3 (alkalische Protease, die ebenfalls in WO 91/02792 und US 5,352,604 beschrieben ist). Es bedeuten D = Asp = Asparaginsäure, R = Arg = Arginin, A = Ala = Alanin, I = Ile = Isoleucin, S = Ser = Serin, T = Thr = Threonin. "BLAP" und dessen Variante mit der Mutation S3T besitzen somit etwa 98 % Homologie zur Aminosäurensequenz der Fig. 1 und werden hier demgemäß auch als "Subtilisin 309"-Typ-Proteasen betrachtet. Bevorzugt betrifft die Erfindung daher hochalkalische Proteasen mit der Dreifachmutation N42R/N114R/N115R, deren zugrundeliegende Aminosäurensequenz mit der in Fig. 1 angegebenen Aminosäurensequenz identisch ist oder sich von dieser entweder nur in Position 85 durch die natürliche Abweichung Asparagin statt Serin oder nur in den folgenden fünf Positionen durch die natürlichen Abweichungen 97D/99R/101A/102I/157S oder nur in den folgenden sechs Positionen durch die natürlichen Abweichungen 3T/97D/99R/101A/102I/157S unterscheidet.

Die den erfindungsgemäßen Proteasen zugrundeliegende alkalische Bacillus-Protease kann aus dem am 28.07.1989 unter der DSM-Nr. 5466 hinterlegten Bacillus-Stamm gewonnen werden (mit der Aminosäurensequenz der Fig. 1) weitere Einzelheiten, insbesondere die Isolierung, zu diesem Stamm sind in der europäischen Patentanmeldung EP 415 296 beschrieben. Die in Position 85 abweichende Variante der den erfindungsgemäßen Protease-Mutanten zugrundeliegenden Protease kann analog durch Kultivierung des Bacillus nov. species PB92 (wie dieses in der europäischen Patentanmeldung EP 283 075 bzw. im hierzu korrespondierenden US-Patent US 5,217,878 beschrieben ist) gewonnen werden. Die in den Positionen 97/99/101/102/157 oder in den Positionen 3/97/99/101/102/157 abweichenden Varianten der den erfindungsgemäßen Protease-Mutanten zugrundeliegenden Protease können analog durch Kultivierung von Bacillus lentus oder nach geeigneter Transformation auch in Bacillus licheniformis (wie dieses in der WO 91/02792 bzw. im hierzu korrespondierenden US-Patent US 5,352,604 beschrieben ist) gewonnen werden. Die Variante in Position 85 sowie die Varianten "BLAP" können aber auch durch Erzeugung entsprechender Punktmutationen in den genannten Positionen aus der Protease mit der in Fig. 1 angegebenen Aminosäurensequenz hergestellt werden. Aus den vorstehenden, den erfindunsgemäßen Protease-Mutanten zugrundeliegenden Proteasen vom "Substilisin 309"-Typ können die erfindungsgemäßen hochalkalischen Proteasen mit der Dreifachmutation N42R/N114R/N115R durch kombinierte oder aufeinanderfolgende Punktmutationen in der Aminosäurensequenz der Vorläuferprotease vom "Subtilisin 309"-Typ in an sich bekannter Weise erzeugt werden. Die Durchführung solcher Punktmutationsverfahren in bezug auf einzelne Aminosäurepositionen ist in der europäischen Patentanmeldung EP 415 296 beschrieben und das dort gegebene Verfahren kann für die Erzeugung der Dreifachmutationen zur Herstellung der erfindungsgemäßen Proteasen analog verwendet werden.

In einer sehr bevorzugten Variante der Erfindung ist die erfindungsgemäße hochalkalische Protease mit der Dreifachmutation N42R/N114R/N115R von einer zugrundeliegenden alkalischen Bacillus-Protease aus dem Stamm DSM 5466 mit einer zugrundeliegenden, zur Fig. 1 identischen Aminosäurensequenz abgeleitet. In den in Kurzschreibweise angegebenen Mutationen beziehen sich die Zahlenangaben auf die Position in der Aminosäurensequenz (siehe Fig. 1). Die ursprüngliche Aminosäure ist der Zahlenangabe vorausgestellt und die durch Mutation in die Aminosäurensequenz in der betreffenden Position eingeführte neue Aminosäure ist der Zahlenangabe nachgestellt. Für die Angabe der Aminosäuren ist der Einbuchstabencode gewählt; N steht hierbei für Asparagin (Asp) und R steht hierbei für Arginin (Arg). Die entsprechenden Aminosäurenaustausche können - wie vorstehend bereits erwähnt - auf an sich bekannte Weise durch Punktmutation in der Aminosäurensequenz erhalten werden, wie dieses Verfahren z. B. in der EP 415 296 beschrieben ist.

Die erfindungsgemäßen hochalkalischen Bacillus-Protease-Mutanten N42R/N114R/N115R zeigen ungewöhnlich gute Aktivität unter den in gewerblichen bzw. industriellen Anwendungen üblichen Bedingungen - wie hohe pH-Werte, hohe Temperaturen, kurze Anwendungszeiten. Auch zeigen die erfindungsgemäßen Protease-Mutanten eine überraschend hohe Beständigkeit gegen die in gewerblichen bzw. industriellen Verfahren - z. B. in gewerblichen Textilwaschverfahren - gebräuchlichen Formulierungsbestandteile. Die erfindungsgemäßen hochalkalischen Bacillus-Protease-Mutanten können daher vorteilhaft in gewerblichen bzw. industriellen Verfahren eingesetzt werden, wie z. B. in gewerblichen Textilwaschverfahren, in jeder Art von gewerblicher Oberflächenreinigung, in Lederbehandlungsverfahren wie insbesondere z. B. in der Lederherstellung. Eine gewerbliche bzw. industrielle Anwendung der erfindungsgemäßen Protease in der gewerblichen Textilwäscherei ist z. B. in der prioritätsgleichen deutschen Patentanmeldung DE 44 11 223 näher beschrieben; so kann die erfindungsgemäße hochalkalische Bacillus-Protease-Mutante z. B. in gewerblichen Großraumtrommelwaschmaschinen oder in vollkontinuierlich oder taktabhängig arbeitenden Gegenstrom-Waschstraßen verwendet werden. Besonders vorteilhaft wird dabei die erfindungsgemäße alkalische Bacillus-Protease bei sogenannten Mehrlaugenverfahren, z. B. Zweibadverfahren aus Vorwasch- und Klarwaschgang, der Flotte im Vorwaschgang zugegeben. Die Vorwäsche kann dabei bei den gewerblichen Textilwaschverfahren üblichen Bedingungen, z. B. bei Temperaturen von 30 bis 70 °C auf an sich bekannte Weise mit den üblicherweise in diesem Waschgang verwendeten Waschmittelinhaltsstoffen durchgeführt werden. Bei stark proteinhaltigen Verunreinigungen, z. B. bei stark blutbefleckter Wäsche aus Krankenhäusern, z. B. Wäsche aus Großküchen oder fleischverarbeitenden Betrieben können die genannten Proteasen gegebenenfalls in einem dem Vorwaschgang vorgeschalteten Vorspülgang mit klarem kalten oder rückgeführtem warmen Wasser und den ansonsten hierfür üblichen Waschmittelinhaltsstoffen mit gutem Erfolg verwendet werden. Weiterhin können die erfindungsgemäßen alkalischen Bacillus-Protease-Mutanten auch in anderen, gewerblichen Textilwaschverfahren, z. B. in auf bestimmte Textil- und Verschmutzungsarten abgestimmten gewerblichen Textilwaschverfahren, wie z. B. bei der desinfizierenden Wäsche von Textilien aus dem Krankenhaus-Sektor, vorteilhaft verwendet werden. Nähere Einzelheiten hierzu finden sich in der DE 44 11 223.

Ein weiteres im Rahmen der vorliegenden Erfindung vorteilhaftes Anwendungsgebiet liegt im Bereich der Reinigung von (harten) Oberflächen in gewerblichen bzw. industriellen Anlagen, vorzugsweise in Schlachtereibetrieben in der Nahrungs- und Genußmittelindustrie, in Großküchen, in Grillbetrieben etc.

Alle Oberflächen, die bei der Herstellung und Weiterverarbeitung sowie beim Transport mit einem Lebensmittel in Berührung kommen, müssen in bestimmten Zeitabständen gereinigt werden. In verschiedenen Branchen (z.B. Getränke-, Konserven-, Zuckerindustrie, milch-, fleisch- und fettverarbeitende Industrie) fallen unterschiedliche Verschmutzungen an. Zur Entfernung dieser Verschmutzungen steht im Stand der Technik ein größeres Sortiment unterschiedlicher Reinigungsmittel zur Verfügung. Diese Reinigungsmittel müssen zunächst Eiweiß-, Fett- und Kohlenhydrat-Verschmutzungen entfernen. Neben den organischen fallen auch anorganische Verschmutzungen an. Die Reinigungsmittel bestehen daher aus einer Mischung verschiedener Komponenten, die beim Reinigungsvorgang bestimmte Funktionen erfüllen. Sie sind als Pulver oder Flüssigkeiten, seltener auch als Pasten käuflich erhältlich und müssen, von wenigen Ausnahmen abgesehen, vom Anwender mit Wasser auf Konzentrationen von 0,5 bis 2 Gew.-% für die Anwendung verdünnt werden. Als Enzyme werden im Bereich der Oberflächenreinigung insbesondere Proteasen und Amylasen für eiweiß- und stärkehaltige Verschmutzungen in speziellen Reinigungsmitteln eingesetzt. Die Anwendung der erfindungsgemäßen Protease-Dreifachmutante in solchen Reinigungsmitteln für die Oberflächenreinigung empfiehlt sich insbesondere dann, wenn die Korrosionsempfindlichkeit des verschmutzten Materials (insbesondere im Falle von Leichtmetallen wie Aluminium und dessen Legierungen) den Einsatz von stärkeren, sauren oder alkalischen Produkten nicht zuläßt, oder beispielsweise auch zur Reinigung von Membranen in Hyperfiltrationsanlagen (Umkehrosmose).

Übliche Inhaltsstoffe solcher speziellen Reinigungsmittel zur Oberflächenreinigung in der Lebensmittelindustrie sind (neben den bereits zuvor erwähnten Enzymen) insbesondere schmutzaufschließende Komponenten, die als Hauptbestandteil solcher Reinigungsmittel zur Ablösung des Schmutzes dienen und meist alkalisch reagierende z.B. Natrium- oder Kaliumhydroxid, Natrium- bzw. Kaliumcarbonat, alkalische Salze der Orthophosphorsäure oder anderer organischer Säuren, sowie Natrium- bzw. Kaliumwasserglassorten mit unterschiedlichen Siliciumdioxid/Alkalioxid-Verhältnissen (SiO₂: Na₂O = 0,7-3,3); weitere Bestandteile sind oberflächenaktive Substanzen, insbesondere zur Beseitigung von fettigen Verschmutzungen (anionische Tenside, z.B. Fettalkoholsulfate, Alkylbenzolsulfonate sowie auch Seifen; außerdem nicht-ionische Fettalkohol- und Alkylphenolglykolether); Korrosionsschutzmittel, insbesondere im Falle von Leichtmetallen wie Aluminium und dessen Legierungen zur Vermeidung der Abtragskorrosion, z.B. im alkalischen Bereich Wasserglas und im sauren Bereich eine Vielzahl spezifisch auf Säure und Material wirkende Inhibitoren; Antischaummittel, z.B. Parafinöl und Silikone, vorzugsweise spezielle Ethoxylierungs- und Propoxylierungs-Produkte.

Als Beispiel für die Verwendung der erfindungsgemäßen Protease-Dreifachmutante auf dem Gebiet der Reinigung von harten Oberflächen seien beispielhaft zwei Reinigungsmittelzusammensetzungen für zwei spezielle Anwendungsfelder genannt:
Grillreiniger:
   - 1,0 Gew.-% Walocel HT 30000 PFV
   - 0,5 Gew.-% Sequinon 40 Na 32
   - 25 Gew.-% Kalilauge (50 Gew.-%)
   - 4 Gew.-% Rewoteric AM VSF
   - 3 Gew.-% Protease-Dreifachmutante
      (Aktivität 300.000 DU/ml, +/- 5 %)
   - 66,5 Gew.-% Wasser
Hochdruckaktivreiniger:
   - 5 Gew.-% Fettalkoholethersulfat (28 gew.-%ig)
   - 10 Gew.-% Sequinon 40 Na 32
   - 10 Gew.-% Natronlauge (50 gew.-%ig)
   - 2 Gew.-% Natrium-Cumolsulfat (40 gew.-%ig)
   - 3 Gew.-% Protease-Dreifachmutante
      (Aktivität 300.000 DU/ml, +/- 5 %).
   - 70 Gew.-% Wasser.

Ein weiteres vorteilhaftes Anwendungsfeld für die erfindungsgemäßen hochalkalischen Protease-Mutanten besteht im Bereich der Lederbehandlung, insbesondere im Bereich der Lederherstellung. Die erfindungsgemäße Protease-Mutante wird dabei insbesondere in Verfahren zur Enthaarung von Fellen oder Häuten eingesetzt, wobei sie vorzugsweise in den Verfahrensschritten Hauptweiche und/oder Äschern Verwendung findet.

Die erfindungsgemäßen Protease-Mutanten eignen sich hervorragend für den Einsatz bei der Lederherstellung. Die Lederherstellung umfaßt unter anderem die Herstellschritte
a) Schmutzweiche zur Entfernung von Salz, Schmutz und Dung
b) Hauptweiche zur Quellung der Haut und Herauslösen wasserlöslicher Proteine;
c) Äscher zur Enthaarung, zum Hautaufschluß und zur weiteren Quellung (Blößen); unter "Blöße" versteht man die dabei erzeugte haarlose, aufgeschlossene Haut.
d) Entkälkung und Beize zum Entfernen von Epidermis- und Hautresten sowie zur Absenkung des pH-Wertes;
e) Pickel zum Sauerstellen der Blößen;
f) Chromgerbung, d. h. eine Mineralgerbung mit Chrom- bzw. Aluminium- oder Zirkonsalzen.

An die vorstehenden Stufen schließt sich im allgemeinen eine Neutralisation, eventuell ein Nachgerben, Färben und Fetten, sowie die Trocknung und Zurichtung des Leders zu verkaufsfähigen Produkten an. Die vorgenannten Arbeiten, außer Trocknung und Zurichtung, werden in der sogenannten Wasserwerkstatt durchgeführt. Als Gefäße verwendet man hierbei Haspel, Fässer und Maschinen nach dem Betonmischer- oder Waschmaschinenprinzip. Enzyme werden in den vorbeschriebenen Arbeitsgängen bis einschließlich des Pickels eingesetzt. Es ist verständlich, daß dabei gerbereichemische Prozesse und andere Anwendungen von proteolytischen Enzymen im pH-Bereich zwischen 3 und 12 unterschiedliche Enzyme in den einzelnen Verfahrensstufen erfordern, da jedes Enzym einen speziellen pH-Aktivitätsbereich besitzt. Die Bandbreite der pH-abhängigen Wirkung eines Enzymproduktes für die Lederherstellung kann erheblich erweitert werden, indem die Enzyme mit verschiedenen pH-Aktivitätsbereichen in Mischungen für das Lederherstellverfahren bereitgestellt werden. Die erfindungsgemäßen Protease-Mutanten lassen sich bei der Lederherstellung - neben gegebenenfalls der Hauptweiche - insbesondere im Verfahrensschritt der Enthaarung (im Äscher) vorteilhaft einsetzen. Die erfindungsgemäßen Mutanten zeichnen sich hierbei dadurch aus, daß sie eine optimale Wirksamkeit im Hinblick auf die Auflösung der für die Haarfixierung verantwortlichen Proteine bis zu einem pH-Wert von pH 12 besonders auch bei den, bei der technischen Enthaarung üblichen, niedrigen Temperaturen (etwa 28 bis 30°C) gewährleisten. Die vorteilhafte, enthaarende Wirkung wird zusätzlich durch eine erhöhte pH-Stabilität der erfindungsgemäßen Protease-Dreifachmutante unterstützt. Von den unter Einsatz der erfindungsgemäßen Protease-Dreifachmutante behandelten Fellen und Häuten lassen sich die Haare sehr leicht entfernen, und es werden hohe Gesamtablösegrade (über ca. 99 %) erhalten. Wie in den Beispielen in einem Labor-Modelversuch gezeigt, bereitet die Ablösung der Haare mit einem Schaber keine Schwierigkeiten. Mit den erfindungsgemäßen Protease-Dreifachmutanten wird daher eine vollkommene und schädigungsfreie enzymatische Enthaarung auch von Großviehhäuten in praxisgerechter Weise ermöglicht. Ferner kann bei Einsatz der erfindungsgemäßen Protease-Dreifachmutanten im Äscher auf umweltbedenkliche Chemikalien (z. B. Sulfid) weitestgehend verzichtet werden.

Die Erfindung betrifft weiterhin ganz allgemein Zusammensetzungen für gewerbliche und/oder industrielle Anwendungen, die die vorstehend beschriebene erfindungsgemäße hochalkalische Protease und für die jeweilige Anwendung an sich übliche weitere Formulierungsbestandteile enthalten. Solche erfindungsgemäßen Zusammensetzungen enthalten z. B. für den Bereich der gewerblichen und/oder industriellen Oberflächenreinigung folgende typische Bestandteile: Alkalien (z.B. NaOH, KOH), anionische und nichtionische Tenside, Komplexierungsmittel, Persauerstoffbleichmittel, Propylenglykol u.a. organische Lösungsmittel, Phosphonate u.a. Builder. Im Bereich der Lederherstellung enthalten typische Zusammensetzungen als Formulierungsbestandteile häufig Natriumsulfat, Ammoniumsulfat, Sägemehl u.a. Bestandteile. Es handelt sich hierbei um feste Produkte, die aus Mischungen von pulverformigen Bestandteilen und Granulaten bestehen.

Neben den vorstehend beschriebenen Vorteilen der erfindungsgemäßen hochalkalischen Bacillus-Protease-Mutanten in gewerblichen und/oder industriellen Anwendungen besitzen die erfindungsgemäßen Protease-Mutanten darüber hinaus auch überraschende Vorteile in Haushaltswasch- und Haushaltsreinigungsmittelzusammensetzungen, die die erfindungsgemäße hochalkalische Protease-Mutante in Kombination mit einer weiteren dem Stand der Technik üblichen Protease vom "Subtilisin 309"-Typ (die nicht die Dreifachmutation N42R/N114R/N115R aufweist). Die erfindungsgemäße hochalkalische Protease-Mutante wirkt sich hierbei in Mischungen mit einer für diesen Haushaltsbereich üblicherweise verwendeten Protease, insbesondere mit einer Protease vom "Subtilisin 309"-Typ, positiv auf die Restwaschleistung nach Lagerung der Haushaltswaschmittel aus. Durch die Kombination der erfindungsgemäßen hochalkalischen Protease wird insbesondere die Lagerstabilität der üblichen Haushaltswaschmittel bzw. Haushaltsreinigungsmittel-Proteasen in Gegenwart von üblichen Wasch- bzw. Reinigungsmittelformulierungsbestandteilen deutlich erhöht. Während Zusammensetzungen mit den im Stand der Technik üblichen Proteasen eine über den beobachtbaren Aktivitätsverlust hinausgehende Verminderung der Waschleistung aufweisen, stellt sich bei Einsatz der erfindungsgemäßen Protease-Dreifachmutante N42R/N114R/N115R im Laufe der im Vertrieb üblichen Lager- und Auslieferungszeiten eine für die Bedingungen von Universalhaushaltswaschmitteln (pH = 9,5 bis 10,5, Waschtemperaturen von insbesondere 30 bis 60 °C) optimale Waschaktivität ein, die die Waschaktivität von Zusammensetzungen, die ausschließlich im Stand der Technik bisher bekannte Proteasen enthalten, übersteigt.

Die Erfindung betrifft daher auch Haushaltswasch- und Haushaltsreinigungsmittelzusammensetzungen, die eine erfindungsgemäße hochalkalische Protease-Mutante in Kombination mit einer weiteren Protease vom "Subtilisin 309"-Typ, die ihrerseits nicht die Dreifach-Mutation N42R/N114R/N115R aufweist, enthalten. Vorteilhafte Haushaltswasch- und Haushaltsreinigungsmittelzusammensetzungen gemäß der Erfindung enthalten die erfindungsgemäße hochalkalische Protease-Mutante in Kombination mit einer weiteren Protease, deren Aminosäurensequenz 95 %, vorzugsweise 98 %, Homologie zu der in Fig. 1 angegebenen Aminosäurensequenz aufweist, wobei die weitere Protease aber nicht die Dreifachmutation N42R/N114R/N115R aufweist. Sehr bevorzugt sind Haushaltswasch- und Haushaltsreinigungsmittelzusammensetzungen, die eine erfindungsgemäße hochalkalische Protease enthalten, deren zugrundeliegende Aminosäurensequenz mit der in Fig. 1 angegebenen Aminosäurensequenz im wesentlichen identisch ist. Bevorzugt sind hierbei solche erfindungsgemäßen hochalkalischen Proteasen mit der Mutation N42R/N114R/N115R, deren zugrundeliegende Aminosäurensequenz mit der in Fig. 1 angegebenen Aminosäurensequenz identisch ist oder die sich von dieser nur in Position 85 durch die natürliche Abweichung Asparagin statt Serin unterscheidet.

Vorzugsweise sollten in den erfindungsgemäßen Anwendungen und Zusammensetzungen solche erfindungsgemäßen alkalischen Bacillus-Protease-Präparate eingesetzt werden, welche eine Enzymaktivität von 50.000 bis 1.000.000 DU/g Enzympräparat aufweisen. Unter "DU" wird dabei die enzymatische Aktivität in Delft Units verstanden, wobei 1.000 DU der proteolytischen Aktivität entsprechen, die bei einem Volumen von 1 ml einer 2 %(W/W)igen Enzymlösung nach Abbau von Kasein eine Extinktionsdifferenz (1 cm Lichtweg; 275 nm; Bestimmung gegen Blindprobentest) von 0,4000 ergibt. Die erfindungsgemäßen alkalischen Bacillus-Protease-Präparationen können dabei in den für gewerbliche bzw. industrielle Verfahren üblichen Formulierungen einzeln oder gewünschtenfalls auch in Kombination mit anderen auf den gewerblichen bzw. industriellen Sektor oder für den Haushaltsbereich gebräuchlichen Proteasen oder anderen an sich üblichen Enzymen, wie z. B. Amylasen, Lipasen, Cellulasen, Pektinasen, Nukleasen, Oxidoreduktasen etc., eingesetzt werden. Bezogen auf die Trockensubstanz der Gesamtzubereitung sollte der Anteil der genannten Bacillus-Proteasen an den erfindungsgemäßen Formulierungen bevorzugt bei 0,1 bis 5 Gew.-%, insbesondere bei 0,2 bis 2 Gew.-% liegen.

Die erfindungsgemäßen Zusammensetzungen können für die verschiedenen Anwendungsfelder auf an sich bekannte Weise in Pulverform, z. B. in Form von Granulaten, Prills oder Pellets, gegebenenfalls auch mit Oberflächenüberzügen (Coating) versehen, konfektioniert sein. Aufgrund der guten Stabilität der erfindungsgemäß genannten Bacillus-Protease-Mutanten können diese auch in Flüssigformulierungen eingesetzt werden.

Bei den für gewerbliche bzw. industrielle Verfahren üblichen Bedingungen - wie hochalkalische pH-Werte, z. B. pH-Werte über 11,0, sowie gegebenenfalls hohe Temperaturen von bis zu 70 °C - zeigt die erfindungsgemäße hochalkalische Bacillus-Protease-Mutante überraschend gute Eigenschaften. Dies ist umso überraschender, als im Vergleich zu üblichen Haushaltsanwendungen in gewerblichen bzw. industriellen Anwendungen zumeist nur relativ kurze Anwendungszeiten zur Verfügung stehen. Neben einer hohen Temperaturbeständigkeit zeigt die erfindungsgemäße hochalkalische Bacillus-Protease-Mutante dabei insbesondere eine hohe Enzymstabilität in Gegenwart gebräuchlicher Inhaltsstoffe für gewerbliche bzw. industrielle Zusammensetzungen oder auch für den Haushaltsbereich. Auch gegen die im gewerblichen bzw. industriellen Sektor oder im Haushaltsbereich gebräuchlichen Bleichmittel, wie sie z. B. in gewerblichen Desinfektions-Waschmitteln für den Krankenhaus-Sektor, bzw. allgemein auch in Haushaltswaschmitteln, eingesetzt werden, ist die erfindungsgemäße hochalkalische Bacillus-Protease-Mutante überraschend stabil.

### Erläuterungen zu den Figuren:

**Figur 1:**
   Sequenzprotokoll der Aminosäurensequenz (SEQ ID NO1) der alkalischen Protease aus Bacillus alcalophilus HA1 (DSM 5466)
**Figur 2:**
   pH-Optimum der Protease-Mutante im Vergleich zur nichtmutierten Ausgangsprotease bei T = 50°C
**Figur 3:**
   Temperatur-Optimum der Protease-Mutante im Vergleich zur nichtmutierten Ausgangsprotease bei pH = 11
**Figur 4:**
   Lagerstabilität der Protease-Mutante im Vergleich zur nichtmutierten Ausgangsprotease in einem Standardwaschmittel (geschlossene Kartons)
**Figur 5:**
   Reinigungsleistung der Protease-Mutante im Vergleich zur nichtmutierten Ausgangsprotease bei pH = 11,4 und T = 15 bis 60°C.

Die Sequenzierung der in Fig. 1 angegebenen Aminosäurensequenz der alkalischen Protease aus Bacillus alcalophilus HA1 (DSM 5466) über Ermittlung der entsprechenden Nukleotidsequenz ist in den Beispielen 1 bis 4 der EP 415 296 beschrieben. Der als Bacillus alcalophilus bezeichnete Stamm HA1 ist am 28.07.1989 bei der Deutschen Sammlung von Mikroorganismen (DSM) unter der Nummer DSM 5466 hinterlegt worden (Anschrift: DMS-Deutsche Sammlung von Mikoorganismen und Zellkulturen GmbH, Mascheroder Weg 1b, D-38124 Braunschweig, Bundesrepublik Deutschland).

### Beispiele

Die folgenden Beispiele sollen die Erfindung erläutern ohne sie jedoch in ihrem Umfang zu beschränken.

### Beispiel 1:

### Herstellung der durch Mutation in der Aminosäurensequenz variierten alkalischen Protease

Die Herstellung der alkalischen Protease, welche sich von der in Fig. 1 angegebenen Aminosäurensequenz der alkalischen Protease aus Bacillus alcalophilus HA1 (DSM 5466) durch die Aminosäurenaustausche N42R, N114R und N115R unterscheidet, wurden auf an sich bekannte Weise durch gerichtete Mutagenese in DNA-Teilsequenzen des entsprechenden Proteasegens durchgeführt. Mit den Zahlenangaben ist dabei die entsprechende Position in der in Fig. 1 angegebenen Aminosäurensequenz bezeichnet, wobei der Positionsangabe im an sich bekannten Einbuchstabencode die ursprüngliche Aminosäure vorangestellt und die eingeführte Aminosäure der Positionsangabe nachgestellt ist. Ausführlich wird für die Einzel-Mutationen das Verfahren der gerichteten Mutagenese in den Beispielen 5 bis 18 der EP 415 296 beschrieben. Zusätzlich sei für die Verfahrensdurchführung zur Erzeugung einzelner Mutationen noch auf die Beispiele der EP 503 346 verwiesen.

Prinzipiell umfaßte das Verfahren die folgenden an sich bekannten Verfahrensschritte:

Aus dem Naturisolat Bacillus alcalophilus HA1 (DSM 5466) wurde nach der Methode von Saito et al. (1963, Biochim. Biophys. Acta 72, 619-629) chromosomale DNA isoliert und mit der Restriktionsendonuklease Sau3A partiell hydrolysiert. Die Restriktionsfragmente wurden durch Elektrophorese aufgetrennt und die Fragmente mit einer Größe von 3 bis 8 Kilobasen isoliert. Die isolierten und größenselektierten DNA-Fragmente aus Bacillus alcalophilus HA1 wurden mit Vektor-DNA des an sich bekannten Plasmids pUB 110 in vitro auf an sich bekannte Weise neu kombiniert. Mit der erhaltenen in vitro neu kombinierten DNA wurden Protoplasten des Stammes Bacillus subtilis BD224 (Bacillus Genetic Stock Center 1A46) nach der von S. Chang und N. Cohen (1979, Mol. Gen. Genet. 168, 111-115) beschriebenen Methode transformiert. Die Transformanten wurden auf Platten mit Neomycin selektiert. Aus einem Klon wurde die Plasmid-DNA nach dem Handbuch von Maniatis et al. (Maniatis et al. = T. Maniatis, E. F. Fritsch, J. Sambrook, Molecular Cloning, A Laboratory Manual, Cold Spring Harbor Laboratory, 1982) isoliert. Das in diesem Plasmid enthaltende Fragment aus der B. alcalophilus-DNA hatte eine Größe von 4,1 KB und enthielt die vollständige DNA-Sequenz für die hochalkalische Protease aus Bacillus alcalophilus HA1 (DSM 5466) (vgl. Beispiel 1 und 2 der EP 415 296).

Das die vollständige DNA-Sequenz für die hochalkalische Protease aus Bacillus alcalophilus HA1 (DSM 5466) enthaltende Plasmid wurde mit AvaI geschnitten. Die überstehenden Enden wurden auf an sich bekannte Weise (Maniatis et al., S. 114) zum DNA-Doppelstrang aufgefüllt. Nach anschließender Restriktion dieser DNA mit XbaI wurde das N-terminale 1.618 BP umfassende Fragment isoliert und auf an sich bekannte Weise in den Vektor pBS kloniert. Der resultierende Vektor enthielt das N-terminale Ende der für die in Fig. 1 abgebildete Aminosäurensequenz codierenden DNA (vgl. Beispiel 5 der EP 415 296).

In analoger Weise wurde ein Vektor erstellt, der ein 658 BP umfassendes für das C-terminales Ende der entsprechenden Protease codierendes DNA-Fragment enthielt. Hierfür wurde das die vollständige DNA-Sequenz enthaltende Plasmid mit den Restriktionsendonukleasen XbaI und Asp718 geschnitten und in die entsprechende Schnittstelle des bekannten Vektors pBS kloniert (vgl. Beispiel 7 der EP 415 296).

Die gerichteten Mutationen wurden in der das C-terminale oder das N-terminale Ende enthaltenden DNA-Teilsequenz mit der von Kunkel, T. A. (1985, Proc. Natl. Acad. Sci. USA 82, 488-492) beschriebenen "primer extension" Technik durchgeführt. Hierzu wurden die entsprechenden Vektoren zunächst in ihre uracilierten einzelsträngigen Analoga auf an sich bekannte Weise umgewandelt, indem mit einem der beiden Vektoren transformierte E. coli CJ236-Bakterien kultiviert wurden, welche zusätzlich mit dem Helfer-Phagen M13 K07 (bezogen von Bio-Rad Laboratories, Richmond, Kalifornien) infiziert wurden. Das Bakterium E. coli CJ236 ist einem an sich bekannten Uracil-N-Glycosylase-Mangelmutante, welche bei der Replikation der Vektoren statt Thymidin das Nukleotid Uracil in die DNA-Sequenz des Vektors einbaut. Uracilierte Vektoren lassen sich auf an sich bekannte Weise vorteilhaft für in vitro-Reaktionen der gerichteten Mutagenese einsetzen, da nach Beendigung der Reaktionen der Uracil-haltige DNA-Einzelstrang, der als Matritze zur Erzeugung mutierter DNA-Stränge diente, durch Behandlung mit Uracil-N-Glycosylase beseitigt werden kann. Der Einsatz der genannten Helfer-Phagen war für die Synthese der Hüllproteine für die gebildete uracilierte einzelträngige Vektor-DNA nötig. Umhüllte uracilierte Einzelstrang-Vektor-DNA wurde aus dem transformierten Wirtsorganismus E. coli CJ236 ausgeschleust und anschließend aus dem Kulturmedium isoliert.

Die isolierten, uracilierten DNA-Einzelstrang-Vektoren des C-terminalen bzw. N-terminalen Endes wurden mit synthetischen Oligonukleotiden hybridisiert, welche die Mutationsstellen enthielten und gleichzeitig als Primer für die nachfolgende Ergänzung zum vollständigen DNA-Doppelstrang mit Mutation dienten. Die hierbei eingesetzten synthetischen Oligonukleotide wurden auf an sich bekannte Weise nach der Methode von Beaucage, S. L. und Caruthers, M. H. (1981, Tetrahedron Letters 22, 1859-1862) hergestellt. Die Synthese des zweiten DNA-Stranges wurde auf an sich bekannte Weise mit Hilfe von T4-DNA-Polymerase und nachfolgender Ligation mit T4-DNA-Ligase durchgeführt (Kunkel et al., 1987, Methods in Enzymol. 154, 367-382). Die gebildete Doppelstrang-Vektor-DNA wurde in E. coli MC 1061 transformiert und die mutierten Vektoren wurden durch Überprüfen der entsprechenden unitären Restriktionsendonukleasen-Erkennungsstellen, die mit den synthetischen Oligonukleotiden eingeführt bzw. entfernt wurden, identifiziert.

Zur Herstellung von z. B. zwei Mutationen entweder im N-terminalen oder im C-terminalen Teil der Protease-DNA wurde das Verfahren nach Einführung einer ersten Mutation in analoger Weise unter Verwendung eines weiteren synthetischen Oligonukleotides zur Einführung einer zweiten Mutation wiederholt.

Es wurden Expressionsvektoren mit Mutationen im C-terminalen Teil bzw. N-terminalen Teil der Protease-DNA-Sequenz hergestellt, indem die durch die gerichtete Mutagenese erhaltenen DNA-Sequenzen mit Restriktionsendonukleasen geschnitten wurden und mit einer Vektor-DNA, welche den entsprechenden anderen terminalen Teil der DNA-Sequenz sowie alle für die Expression notwendigen Elemente enthielt, ligiert wurden. Die erhaltenen Vektoren stellten vollständige Expressionsvektoren mit geeignetem Leseraster zur Expression der entsprechend mutierten Protease dar. Die Herstellung von Expressionsvektoren dieser Art erfolgte analog der ausführlich in Beispiel 16 der EP 415 296 beschriebenen Methode. Für die folgende Dreifachmutation wurde der Expressionsvektor DSM 5466 Mut. N42R/N114R/N115R analog hergestellt.

Die dreifach mutierte hochalkalische Protease wurde hergestellt, indem B. subtilis BD 224 mit dem obengenannten Expressionsvektor auf an sich bekannte Weise transformiert und kultiviert wurde. Aus den Kulturüberständen des transformierten und kultivierten Stammes wurde die dreifach mutierte hochalkalische Protease nach bekannten Verfahren isoliert. Ausführliche Angaben zur Isolierungsmethode von mutierten Proteasen finden sich in den Beispielen 16 und 18 der EP 415 296, nach denen hier analog gearbeitet wurde.

Die durch Dreifachmutation in der Aminosäurensequenz variierte alkalische Protease auf Grundlage des Bacillus alcalophilus HA1 (DSM 5466) wurde in den in Beispiel 2 bis 4 beschriebenen Versuchen eingesetzt.

Die Anwendung und die Vorteile der erfindungsgemäßen Dreifachmutante im Bereich der gewerblichen Textilwäscherei sind in den Beispielen der parallelen und hier prioritätsbegründenden deutschen Patentanmeldung DE 44 11 223 ausführlich beschrieben, worauf hier daher nur verwiesen wird.

Weitere Eigenschaften der erfindungsgemäßen Protease-Mutante sind im Beispiel 5 angegeben.

### Beispiel 2:

### Bestimmung der Enthaarungseigenschaft der erfindungsgemäßen Protease-Dreifachmutante

### Vorreinigung:

Rinderhaut 1 h gewässert in doppelter Wassermenge (bezogen auf Rinderhaut) mit 0,1 % Marlipal 013/939 bei ca. 28-30 °C. Dann Rinderhaut in Stücke von ca. 20 x 60 cm zugeschnitten und bei -20 °C gelagert. Aus den Lagerstücken werden Teststücke von ca 20 x 7 cm zugeschnitten, diese läßt man abtropfen und troknet sie mit Papierhandtücher ab. Die Teststücke werden für 120 min. in einer Lösung mit 0,5 % Soda und 0,1 % Marlipal inkubiert und 15 min. umgerührt. Die Gewichtszunahme wird protokolliert.

### A. Quellung (Wasseraufnahme) der vorgereinigten Häute

- Inkubationslösung:: 0,5 % Na-Carbonat, 0,1 % Marlipal (bez. auf die Haut), doppelte Wassermenge (bez. auf die Haut
- Inkubationszeit:: 120 min.
- Inkubationstemperatur:: 28 °C
- Enzym:: Optimase L660

| **Test** | **Enzymdosierung:** | | **Gewicht nach Inkubation** | **Gewicht vor Inkubation** | **Differenz Gewichtszunahme:** | |
|---|---|---|---|---|---|---|
| | **[g/100kg Haut]** | **[AADU/kg]** | **[g]** | **[g]** | **[g]** | **[%]** |
| 1 | 6,08 | 37470 | 729,53 | 701,59 | 27,940 | 4,0 |

### B. Enthaarung

- Inkubationslösung:: 7,1 g/l Ca(OH)₂ (200 % bez. auf Rinderhaut)
- Inkubationszeit:: 24 h
- Inkubationstemperatur:: 30 °C
- Durchmischung:: Zu Beginn der Inkubation 2 h permanent in Linitest-Maschine und zum Ende 3,5 h permanent.
- pH-Wert:: Anfang: 12,48 Ende: 12,45

| **Becher- Nr.** | **Protease** | **Enzymdosierung** | | **Ablösung der Haut *** |
|---|---|---|---|---|
| | | **[g/100 kg Haut]** | **[AADU/kg Haut]** | |
| 1 | Blindtest | -- | -- | 3 |
| 2 | Blindtest | -- | -- | 3 |
| 3 | Mutante | 310,81 | 925188 | 1 |
| 4 | Mutante | 310,81 | 925188 | 1 |

| | | | | |
|---|---|---|---|---|
| Mutante = eingesetzte Protease-Dreifachmutante N42R/N114R/N115R mit Aktivität 300.000 DU/ml (+/- 5%) * 1: leichte und vollständige Entfernung der Haare 2: fast vollständige Entfernung der Haare, aber noch (klumpige) Rückstände auf der Haut 3: es lösen sich nur sehr wenige bis gar keine Haare ab Kommentar: Nach dem Arbeitsschritt A waren die Teststücke weich und schwammig, nach dem Arbeitsschritt B waren die Teststücke "gummiartig ausgehärtet". | | | | |

### Beispiel 3:

### Herkunft der Teststücke: Kuh, Rückenpartie

### Vorreinigung:

Haut 1 h gewässert in doppelter Wassermenge (bezogen auf Haut) mit 0,1 % Marlipal 0 13/939 bei ca 28 - 30 °C. Dann Haut in Stücke von ca 20 x 60 cm zugeschnitten und bei -20 °C gelagert. Aus den Lagerstücken werden Teststücke von ca. 20 x 7 cm zugeschnitten, diese läßt man abtropfen und trocknet sie mit Papierhandtücher ab. Die Teststücke werden für 120 min. in einer Lösung mit 0,5 % Soda und 0,1 % Marlipal inkubiert und 15 min. umgerührt. Die Gewichtszunahme wird protokolliert.

Es wurden 3 Tests unter den nachfolgend angegebenen Bedingungen durchgeführt.

### A. Quellung (Wasseraufnahme) der vorgereinigten Häute

Inkubationslösung: 0,5 % Na-Carbonat, 0,1 % Marlipal (bez. auf die Haut), doppelte Wassermenge (bez. auf die Haut)
Inkubationszeit: 120 min.
Inkubationstemperatur: 28 °C
Enzym: Optimase L660

| **Test** | **Enzymdosierung:** | | **Gewicht nach Inkubation** | **Gewicht vor Inkubation** | **Differenz Gewichtszunahme:** | |
|---|---|---|---|---|---|---|
| | **[g/100kg Haut]** | **[AADU/kg]** | **[g]** | **[g]** | **[g]** | **[%]** |
| 1** | 15,89 | 20060 | 715,98 | 732,58 | - 16,60 | - 2,3 |
| 2 | 3,26 | 20122 | 905,27 | 866,22 | 39,05 | 4,5 |
| 3 | 3,32 | 20476 | 338,39 | 342,25 | - 3,86 | - 1,1 |

| | | | | | | |
|---|---|---|---|---|---|---|
| ** Die Teststücke haben 1,5 h im Kühlschrank gelegen, zwischen Schritt A und B. | | | | | | |

### B. Enthaarung

Inkubationslösung: 7,1 g/l Ca(OH)₂ (200 % bez. auf Haut)
Inkubationszeit: 24 h
Inkubationstemperatur: 30 °C
Durchmischung: Zu Beginn der Inkubation 2 h permanent in Linitest-Maschine und zum Ende 3,5 h permanent.
pH-Wert:

| | Anfang: | Ende: |
|---|---|---|
| Test 1 | 12,48 | 12,46 |
| Test 2 | 12,55 | 12,53 |
| Test 3 | 12,5 | 12,5 |

### Ergebnisse Test 1:

Eingesetzte Protease: Mutante N42R/N114R/N115R mit Aktivität 300.000 DU/ml (+/- 5%)

| **Becher- Nr.** | **Enzymdosierung** | | **Ablösung der Haut *** |
|---|---|---|---|
| | **[g/100 kg Haut]** | **[AADU/kg Haut]** | |
| 1 | -- | -- | 3 |
| 2 | -- | -- | 3 |
| 3 | 31,2 | 92745 | 1,4 |
| 4 | 31,2 | 92745 | 1,2 |
| Kommentar: Die Haare wurden von der Länge her gleichmäßig abgelöst, es blieben jedoch ca. 1 - 3 mm Stoppeln stehen. | | | |

### Ergebnisse Test 2

Eingesetzte Protease: Mutante N42R/N114R/N115R mit Aktivität 300.000 DU/ml (+/- 5%)

| **Becher- Nr.** | **Enzymdosierung** | | **Ablösung der Haut *** |
|---|---|---|---|
| | **[g/100 kg Haut]** | **[AADU/kg Haut]** | |
| 1 | 10,1 | 29969 | 2,2 |
| 2 | 50,3 | 149844 | 1,8 |
| 3 | 100,7 | 299688 | 2,0** |
| 4 | 151,0 | 449531 | 1,4** |

| | | | |
|---|---|---|---|
| ** Nackenpartien mit festerem Haarwuchs | | | |

### Ergebnisse Test 3

Eingesetzte Protease: Mutante N42R/N114R/N115R mit Aktivität 300.000 DU/ml (+/- 5%)

| **Becher- Nr.** | **Enzymdosierung** | | **Ablösung der Haut *** |
|---|---|---|---|
| | **[g/100 kg Haut]** | **[AADU/kg Haut]** | |
| 1 | 151,3 | 450294 | 1,2 |
| 2 | 100,9 | 300196 | 1,4 |
| 3 | 50,4 | 150098 | 1,6 |

| | | | |
|---|---|---|---|
| *** Legende zu Test 1 bis 3** 1: leichte und vollständige Entfernung der Haare 2: fast vollständige Entfernung der Haare, aber noch (klumpige) Rückstände auf der Haut 3: es lösen sich nur sehr wenige bis gar keine Haare ab | | | |

### Beispiel 4:

### A. Hauptweiche

Rinderhaut (Gewicht): 1886,25 [g] (Vorreinigung wie in Beispiel 2 und 3 angegeben)
Körperpartie: Bauch
Ansatz:

| | |
|---|---|
| Wasser | 3772,50 [g] |
| Na₂CO₃ | 9,43 [g] |
| Tensid | 1,89 [g] Marlipal |
| Enzym | 0,3046 [g] Optimase L660 |
| entspricht | 99582 [AADU/kg Rinderhaut] |

Gewicht nach 4 h Reaktion: 2039,5 [g]
Gewichtszunahme: 8,1 [Gew.-%]
pH-Wert nach 4 h Reaktion: 10,21

### B. Enthaarung

- Wasser:: 3059,25 [g]
- Ca(OH)₂: 20,40 [g]
- Enzym:: 6,9352 [g] Protease-Mutante mit Aktivität = 150.000 DU/ml (+/- 5%)
- entspricht:: 539878 [AADU/kg Rinderhaut]
- pH-Wert nach 17,25 h Inkubation:: 12,75

### Optische Beurteilung der Enthaarung:

Die Haare ließen sich sehr leicht entfernen, beim Herausnehmen des Teststückes aus der Trommel wurden die Haare schon durch die Berührung des Öffnungsrandes entfernt. Es blieben nach 2 - 3 Stellen je ca. 2 cm², machte mit dem Schaber keinerlei Schwierigkeiten.

### Beispiel 5:

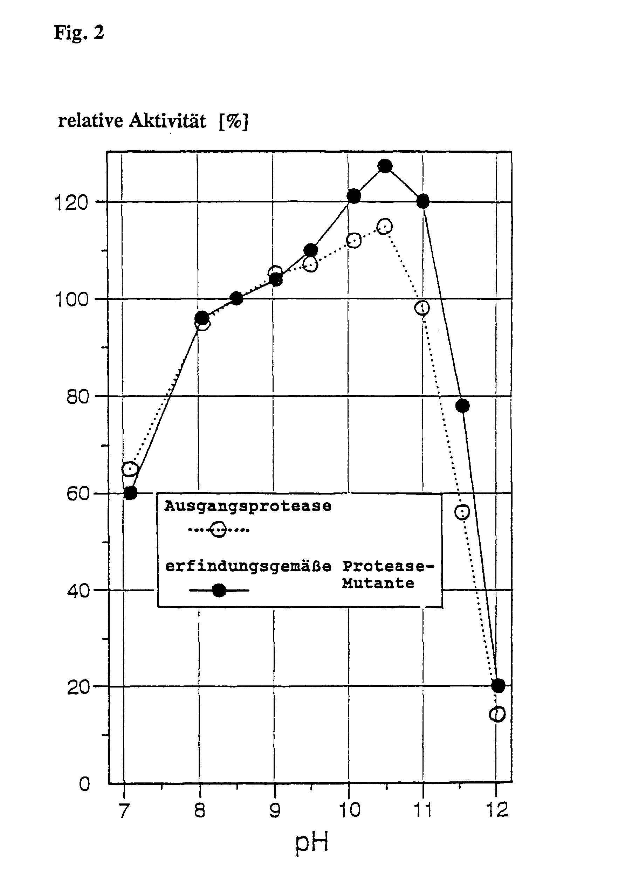

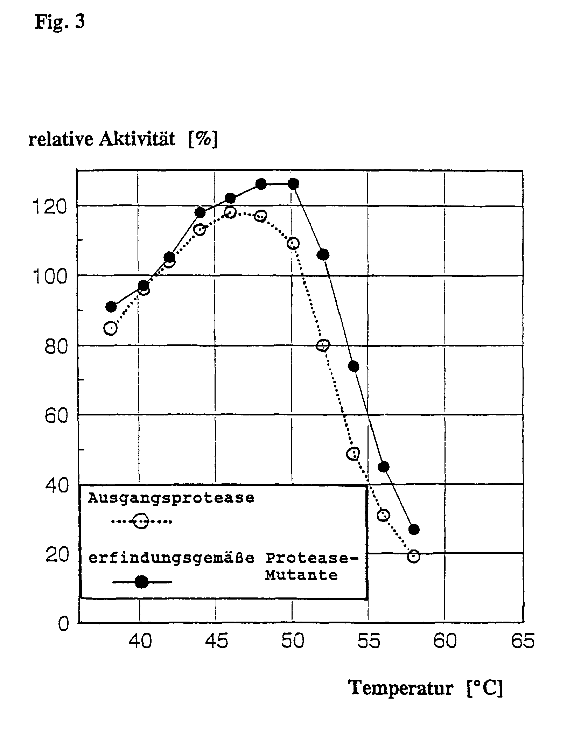

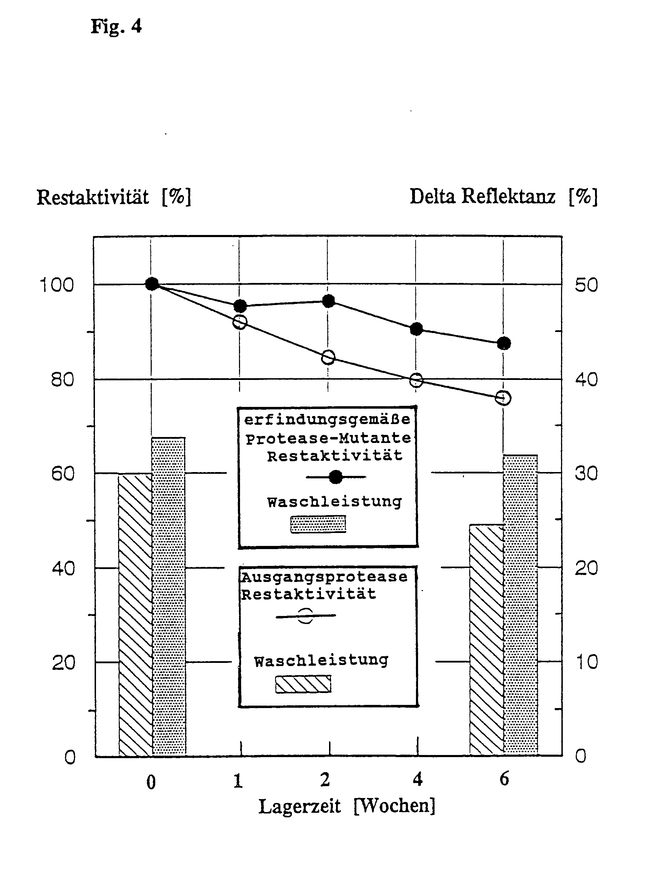

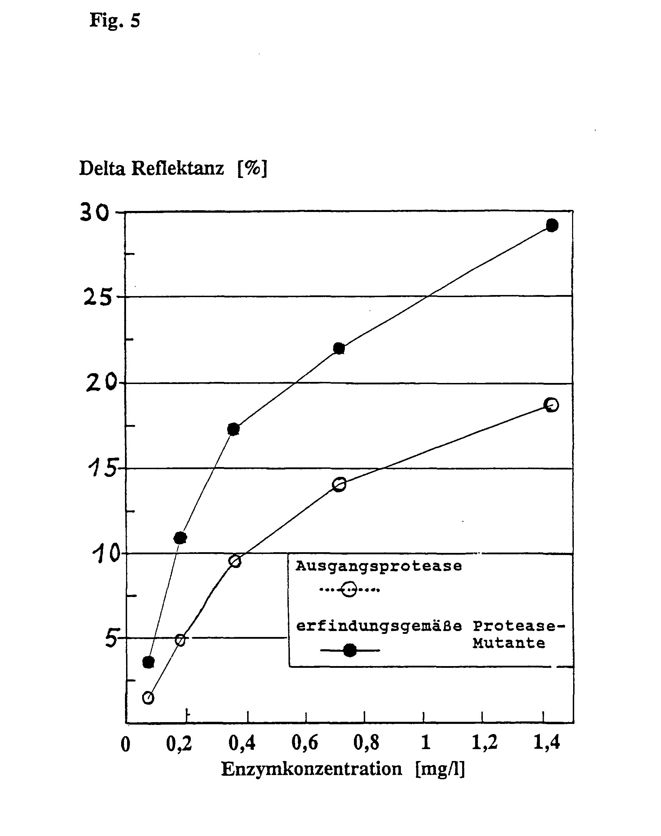

Im nachfolgenden werden noch einige wesentliche Eigenschaften der erfindungsgemäßen Dreifachmutante erläutert.
a) Die Aktivität der erfindungsgemäßen Dreifachmutante im Vergleich zur nichtmutierten Ausgangsprotease in Abhängigkeit vom pH-Wert wurde unter folgenden Bedingungen bestimmt: Substrat = Acetylkasein, T = 50 °C, Umsetzungsdauer = 10 min, Phosphat-Borat-Puffer. Die gefundene pH-abhängige relative Aktivität in % (Aktivität bei pH = 8,5 als 100 % definiert) ist in Fig. 2 wiedergegeben. Die erfindungsgemäße Dreifachmutante zeigt gegenüber der Ausgangsprotease deutlich erhöhte Aktivität bei höheren pH-Werten im Bereich pH = 10 bis 12.
b) Die Aktivität der erfindungsgemäßen Dreifachmutante im Vergleich zur nichtmutierten Ausgangsprotease in Abhängigkeit von der Temperatur wurde unter folgenden Bedingungen bestimmt: Substrat = Acetylkasein, pH = 11. Die gefundene temperaturabhängige relative Aktivität in % (Aktivität bei pH = 8,5 und T = 50 °C als 100 % definiert) ist in Fig. 3 wiedergegeben. Die erfindungsgemäße Dreifachmutante zeigt beim gewählten hohen pH-Wert deutlich verbesserte Aktivität gegenüber der Ausgangsprotease im Temperaturbereich von 45 bis 58 °C.
c) Die Lagerstabilität (shelf life) der erfindungsgemäßen Dreifachmutante im Vergleich zur nichtmutierten Ausgangsprotease in Abhängigkeit von der Lagerzeit wurde durch Lagerung in einem Standardvollwaschmittel unter folgenden Bedingungen bestimmt: T = 30 °C, 60 % relative Feuchtigkeit (r.H. = relative Humidity), in geschlossenen Kartons. Die nach Lagerung gefundene Restaktivität in % (bezogen auf die Anfangsaktivität = 100 %) weist für die erfindungsgenmäße Dreifachmutante eine gegenüber der Ausgangsprotease deutlich höhere Restaktivität und somit verbesserte Stabilität aus. Die Ergebnisse sind in Fig. 4 verdeutlicht.
d) Zur Demonstration der guten Reinigungswirkung (Oberflächenreinigung) der erfindungsgemäßen Dreifachmutante wurde die Waschleistung gegenüber der Ausgangsprotease für verschiedene Enzymkonzentrationen unter folgenden Bedingungen bestimmt: Standard-Detergenz-Basis; pH = 11,4 (6 g/l); Test-Gewebe = EMPA 117, T = 60 °C. Die gefundenen Ergebnisse (Delta Reflektanz) in Abhängigkeit zur eingesetzten Enzymmenge (mg/l) sind in Fig. 5 verdeutlicht, die die Überlegenheit der erfindungsgemäßen Protease bei hohen pH und erhöhter Temperatur ausweist.
e) Zur Demonstration der vorteilhaften Kombination der erfindungsgemäßen Dreifachmutante mit Waschmittelproteasen des Standes der Technik (hier die Ausgangsprotease vom "Subtilisin 309"-Typ) wurden die nachfolgenden beiden Versuche mit den angegebenen Ergebnissen durchgeführt.

### Versuch 1

Relative Waschleistung der erfindungsmäßen Dreifachmutante im Vergleich zur nichtmutierten Ausgangsprotease (jeweils mit Coating versehen) vor und nach Lagerung in einen Universalvollwaschmittel für den Haushaltsbereich. Es handelt sich um die mittlere Waschleistung aus 2 Testgeweben bei aktivitätsgleicher Dosierung.

| **Protease** | **vor Lagerung** | **nach 6 Wochen Lagerung bei 35°C/80% r.H.** |
|---|---|---|
| Ausgangsprotease | 97,15 | 28,15 |
| Dreifachmutante N42R/N114R/N115R | 66,22 | 50,72 |
| | 61,60 | 50,79 |

### Versuch 2

Restaktivität und Restwaschleistung nach Lagerung der erfindungsgemäßen Dreifachmutante bzw. Ausgangsprotease in einem Pulverwaschmittel bei 30° C und 60 % r.H (r.H. = relative Humidity = relative Feuchte); pH-Wert vor dem Waschen jeweils 10,2.

| **Lagerzeit** | **A** | **B** | **C** | **D** |
|---|---|---|---|---|
| 0 Wochen | 100,0 | 24,50 | 100,0 | 22,99 |
| 1 Woche | 92,4 | -- | 100,0 | -- |
| 2 Wochen | 86,9 | -- | 94,6 | -- |
| 4 Wochen | 75,1 | -- | 86,0 | -- |
| 6 Wochen | 60,9 | 14,98 | 70,8 | 21,63 |

- Testgewebe:: Blut/Milch/Tusche (EMPA117)
- % dR:: Differenz der Reflektion des Testgewebes (enzymhaltiges Waschmittel) zu der Reflektion des Testgewebes gewaschen mit der enzymfreien Basis
- Enzymdosierung:: aktivitätsgleich

A = Restaktivität [%], Ausgangsprotease
B = Waschleistung [% dR], Ausgangsprotease
C = Restaktivität [%], Dreifachmutante
D = Waschleistung [% dR], Dreifachmutante

### Definitionen

- Optimase L660 =: alkalische Protease aus B. licheniformis
- Marlipal =: Polyethylenglykol-isotridecyl-ether
- Walocel =: 2-Hydroxyethyl-celluloseether
- Sequinon =: [[Bis[2-[bis(phosphonomethyl)amino]ethyl]amino]methyl]-phosponsäure, Natriumsalz
- Rewoteric =: Ampholyt auf Imidazolinbasis
- AADU =: Autoanalyzer Delf Units
- EMPA117 =: Testgewebe Polyester/Baumwolle mit Anschmutzung Blut/Milch/Tusche (Eidgenössische Materialprüfungsanstalt, Schweiz)

## Patentansprüche

1. Hochalkalische Protease, **gekennzeichnet durch** eine zugrundeliegende Aminosäurensequenz, welche mindestens 95 % Homologie zu der in Fig. 1 angegebenen Aminosäurensequenz aufweist und sich von dieser **durch** Dreifachmutation in den Positionen 42/114/115 der Fig. 1 oder in den drei jeweils dazu homologen Positionen **dadurch** unterscheidet, daß die in den betreffenden Positionen befindlichen Aminosäuren gegen Arginin ausgetauscht sind, worin die Zahl der in den Sequenzen miteinander übereinstimmenden Aminosäuren ("homologe Positionen") bezogen auf die Gesamtzahl der in der Sequenz der Fig. 1 enthaltenen Aminosäuren die Homologie in % angibt.

2. Hochalkalische Protease nach Anspruch 1, **gekennzeichnet durch** eine zugrundeliegende Aminosäurensequenz, welche mindestens 98% Homologie zu der in Fig. 1 angegebenen Aminosäurensequenz aufweist und die sich von dieser **durch** Dreifachmutation in den Positionen 42/114/115 der Fig. 1 **dadurch** unterscheidet, daß die in den betreffenden Positionen befindlichen Aminosäuren gegen Arginin ausgetauscht sind.

3. Hochalkalische Protease nach Anspruch 2, **dadurch gekennzeichnet, daß** die zugrundeliegende Aminosäurensequenz mit der in Fig. 1 angegebenen Aminosäurensequenz identisch ist oder sich von dieser entweder nur in Position 85 durch die natürliche Abweichung Asparagin statt Serin oder nur in den folgenden Positionen durch die natürlichen Abweichungen 97D/99R/101A/102I/157S oder die natürliche Abweichungen 3T/97D/99R/101A/102I/157S unterscheidet.

4. Verwendung der hochalkalischen Protease gemäß einem der Ansprüche 1 bis 3 in gewerblichen oder industriellen Anwendungen.

5. Verwendung nach Anspruch 4 von hochalkalischen Proteasen gemäß einem der Ansprüche 1 bis 3 zur Reinigung von Oberflächen in gewerblichen oder industriellen Anlagen, vorzugsweise in Schlachtereibetrieben, in der Nahrungs- und Genußmittelindustrie, in Großküchen, in Grillbetrieben.

6. Verwendung nach Anspruch 5 von hochalkalischen Proteasen gemäß einem der Ansprüche 1 bis 3 in Verfahren zur Lederherstellung, insbesondere in Verfahren zur Enthaarung von Fellen oder Häuten.

7. Zusammensetzungen für gewerbliche und/oder industrielle Anwendungen, enthaltend eine hochalkalische Protease gemäß einem der Ansprüche 1 bis 3 und für die jeweilige Anwendung an sich übliche weitere Formulierungsbestandteile.

8. Haushaltswasch- und Haushaltsreinigungsmittelzusammensetzung, enthaltend eine hochalkalische Protease gemäß einem der Ansprüche 1 bis 3 in Kombination mit einer weiteren Protease, deren Aminosäurensequenz 95 %, vorzugsweise 98 %, Homologie zu der in Fig. 1 angegebenen Aminosäurensequenz aufweist, wobei die weitere Protease aber nicht die Dreifachmutation N42R/N114R/N115R aufweist.

9. Haushaltswasch- und Haushaltsreinigungsmittelzusammensetzung nach Anspruch 8, enthaltend eine hochalkalische Protease gemäß einen der Ansprüche 1 bis 3 in Kombination mit einer weiteren Protease, deren Aminosäuresequenz identisch zu der in Fig. 1 angegebenen Aminosäurensequenz ist oder sich von dieser entweder nur in Position 85 durch die natürliche Abweichung Asparagin statt Serin oder nur in den folgenden Positionen durch die natürlichen Abweichungen 97D/99R/101A/102I/157S oder die natürliche Abweichungen 3T/97D/99R/101A/102I/157S unterscheidet.

10. Haushaltswasch- und Haushaltsreinigungsmittelzusammensetzung nach Anspruch 8, enthaltend eine hochalkalische Protease gemäß Anspruch 3 in Kombination mit einer weiteren Protease, deren Aminosäuresequenz identisch zu der in Fig. 1 angegebenen Aminosäurensequenz ist oder sich von dieser nur in Position 85 durch die natürliche Abweichung Asparagin statt Serin unterscheidet.

## Claims

1. High-alkaline protease, **characterized by** an underlying amino acid sequence which is at least 95% homologous to the amino acid sequence indicated in Fig. 1 and which differs from the latter by triple mutation in positions 42/114/115 of Fig. 1 or in the three positions in each case homologous thereto in that the amino acids in the positions in question have been replaced with arginine, in which the number of the amino acids corresponding to one another in the sequences ("homologous positions") indicates the homology in %, based on the total number of amino acids comprised in the sequence of Fig. 1.

2. High-alkaline protease according to Claim 1, **characterized by** an underlying amino acid sequence which is at least 98% homologous to the amino acid sequence indicated in Fig. 1 and which differs from the latter by triple mutation in positions 42/114/115 of Fig. 1 in that the amino acids in the positions in question have been replaced with arginine.

3. High-alkaline protease according to Claim 2, characterized i n that the underlying amino acid sequence is identical to the amino acid sequence indicated in Fig. 1 or differs from the latter either only in position 85 by the natural variation of asparagine for serine or only in the following positions by the natural variations 97D/99R/101A/102I/157S or the natural variations 3T/97D/99R/101A/102I/157S.

4. Use of the high -alkaline protease according to any of Claims 1 to 3 in commercial or industrial applications.

5. Use according to Claim 4 of high -alkaline proteases according to any of Claims 1 to 3 for cleaning surfaces in commercial or industrial plants, preferably in abattoirs, in the food and luxury food industries, in canteen kitchens, in rotisseries.

6. Use according to Claim 5 of high -alkaline proteases according to any of Claims 1 to 3 in processes for producing leather, in particular in processes for removing hair from hides and skins.

7. Compositions for commercial and/or industrial applications, comprising a high -alkaline protease according to any of Claims 1 to 3 and further formulation ingredients customary per se for the particular application.

8. Domestic detergent composition and domestic cleaner composition, comprising a high -alkaline protease according to any of Claims 1 to 3 combined with another protease whose amino acid sequence is 95%, preferably 98%, homologous to the amino acid sequence indicated in Fig. 1 but which does not have the triple mutation N42R/N114R/N115R.

9. Domestic detergent composition and domestic cleaner composition according to Claim 8, comprising a high-alkaline protease according to any of Claims 1 to 3 combined with another protease whose amino acid sequence is identical to the amino acid sequence indicated in Fig. 1 or differs from the latter either only in position 85 by the natural varia tion of asparagine for serine or only in the following positions by the natural variations 97D/99R/101A/102I/157S or the natural variations 3T/97D/99R/101A/102I/157S.

10. Domestic detergent composition and domestic cleaner composition according to Claim 8, comprising a high-alkaline protease according to Claim 3 combined with another protease whose amino acid sequence is identical to the amino acid sequence indicated in Fig. 1 or differs from the latter only in position 85 by the natural variation of asparagine for serine.

## Revendications

1. Protéase à forte teneur alcaline, **caractérisée par** une séquence d'acides aminés de base qui présente au moins 95% d'homologie avec la séquence d'acides aminés indiquée à la figure 1 et se différencie de celle-ci par mutation triple aux positions 42/114/115 de la figure 1 ou aux trois positions qui leur sont homologues par le fait que, dans les positions correspondantes, les acides aminés qui s'y trouvent ont été remplacés par l'arginine et en ce que le nombre des acides aminés correspondants les uns aux autres dans les séquences (positions homologues) rapporté au nombre total des acides aminés contenus dans la séquence de la figure 1 indique l'homologie en %.

2. Protéase à forte teneur alcaline selon la revendication 1, **caractérisée par** une séquence d'acides aminés de base qui présente au moins 98% d'homologie avec la séquence d'acides aminés indiquée à la figure 1 et qui se différencie de celle-ci par une mutation triple aux positions 42/114/115 de la figure 1 par le fait que les acides aminés se trouvant aux positions correspondantes sont échangées par l'arginine.

3. Protéase à forte teneur alcaline selon la revendication 2, **caractérisée en ce que** la séquence d'acides aminés de base est identique à la séquence d'acides aminés indiquée à la figure 1, ou bien se différencie de celle-ci soit uniquement à la position 85 par le remplacement naturel de l'Asparagine à la place de la sérine ou uniquement aux positions suivantes par les remplacements naturels 97D/99R/101A/102I/157S ou les remplacements naturels 3T/97D/99R/101A/102I/157S.

4. Utilisation de la protéase à forte teneur alcaline selon l'une quelconque des revendications 1 à 3 dans des utilisations commerciales ou industrielles.

5. Utilisation selon la revendication 4 des protéases à forte teneur alcaline selon l'une quelconque des revendications 1 à 3 pour le nettoyage des surfaces dans des installations commerciales ou industrielles, avantageusement dans les abattoirs dans l'industrie de l'alimentation et des produits de consommat ion de luxe dans les grandes cuisines et dans les grills.

6. Utilisation selon la revendication 5 de protéase à forte teneur alcaline selon l'une quelconque des revendications 1 à 3 dans des procédés pour la fabrication du cuir, en particulier dans des p rocédés pour l'épilation des fourrures ou peaux.

7. Compositions pour des utilisations commerciales et/ou industrielles, contenant une protéase à forte teneur alcaline selon l'une quelconque des revendications 1 à 3, et pour toute utilisation d'autres co nstituants habituels de la formulation.

8. Compositions pour le lavage domestique et le nettoyage domestique contenant une protéase à forte teneur alcaline selon l'une quelconque des revendications 1 à 3 en combinaison avec une autre protéase, dont la séquence d'acides aminés présente une homologie de 95%, avantageusement 98%, avec la séquence d'acides aminés indiquée à la figure 1, l'autre protéase ne présentant cependant pas la triple mutation N42R/N114R/N115R.

9. Composition pour le lavage domestique et le nettoyage domestique selon la revendication 8 contenant une protéase à forte teneur alcaline selon l'une quelconque des revendications 1 à 3 en combinaison avec une autre protéase dont la séquence d'acides aminés est identique à la séquence d'acides aminés indiquée à la figure 1 ou ne se différencient de celle-ci qu'à la position 85 par l'échange naturel de l'asparagine à la place de la sérine ou uniquement aux positions suivantes par les échanges naturels 97D/99R/101A/102I/157S ou les échanges naturels 3T/97D/99R/ 101A/102I/157S.

10. Composition d'un agent pour le lavage domestique et le nettoyage domestique selon la revendication 8, contenant une protéase à forte teneur alcaline selon la revendication 3, en combinaison avec une autre protéase dont la séquence d'acides aminés est identique à la séquence d'acides aminés indiquée à la figure 1 ou ne se différencie de celle-ci qu'à la position 85 par l'échange naturel de l'asparagine à la place de la sérine.
